# EUROPEAN PATENT SPECIFICATION

(11) **EP 1 803 428 B1**
(45) Date of publication and mention of the grant of the patent: **12.01.2011**
(21) Application number: 05113019.3
(22) Date of filing: 28.12.2005
(51) Int. Cl.: A61F 13/15, A61F 13/494, A61F 13/495

(54) **Absorbent articles with comfortable elasticated laminates**
Absorbierende Artikel mit elastischen komfortablen Laminaten
Articles absorbants avec des laminés élastiques et comfortables

(43) Date of publication of application: 04.07.2007
(73) Proprietor: The Procter and Gamble Company, Cincinnati, OH 45202 (US)
(72) Inventor: Arizti, Blanca, 60325, Frankfurt am Main (DE); Baquer-Moras, Gemma, 65824 Schwalbach (DE); Ponomarenko, Ekaterina, 65812 Bad Soden am Taunus (DE); Seeboth (née Brinkmann), Simone, 65824 Schwalbach (DE); Dahlinger, David, Mason, OH 45040 (US); Tucker, David, Mason, OH 45040 (US); Greer, D. Randell, Lockland, OH 45215 (US)
(74) Representative: McGregor, Judit Ester

(56) References cited:
- EP-A- 0 486 006
- EP-A- 1 101 476
- EP-A- 1 166 728
- EP-A- 1 226 801
- EP-A- 1 520 569
- WO-A-03/059603
- US-A1- 2005 084 656

## Description

### FIELD OF THE INVENTION

This invention relates to disposable absorbent articles comprising a topsheet (20) that comprises one or more elastic laminate portion(s) (10) with substantially y-direction elongation (stretch), formed from a topsheet material (supporting material), and an elastic material (12) (and optionally an attachment means and/ or attachment sheet and/ or cover strip (13)), whereby said elastic laminate portions (10) have wrinkles of a very low wrinkle height and a selected average wrinkle density, in order to reduce the risk of severe pressure marks. The elastic laminate portion(s) (10) are preferably very thin. The provision of such elastic laminate portions (10) still provides an excellent force profile for the topsheet (20) and the absorbent article as a whole. Overall, comfortable-to-wear absorbent articles are obtained that provide excellent exudates isolation away from the skin whilst causing very little or no pressure marks in use.

### BACKGROUND TO THE INVENTION

Absorbent articles such as infant diapers, training pants and adult incontinence garments typically comprise elastic leg cuffs and or barrier cuffs to reduce leakage of exudates from the article. Often, they also comprise an elasticated waist band, to improve the fit and comfort when the wearer is moving. A certain type of diapers for feces isolation comprises (also) a topsheet with a large opening with there along elastic bands to ensure correct alignment of the topsheet and the opening with the anus of the user, and to thus ensure optimum reception and isolation of the feces under the topsheet.

These elasticated portions of such articles typically comprise an elastic material laminated to a non-elastic sheet, such as a plastic film, or nonwoven material, obtained by attaching the elastic material in stretched state to the sheet. The resulting laminate thus comprises in unstretched, contracted state and in partially stretched state a surplus of sheet material that forms wrinkles.

Such elasticated portions of the diaper can be uncomfortable in use, due to the pressure of the elastic portions on the skin and/ or due to rubbing of the wrinkled elasticated portions over the skin.

The inventors have also found that even if the user does not experience the elasticated portions as uncomfortable, the red skin marks caused by the elasticated portions may still be perceived by the care taker as uncomfortable for the user.

A prior art document is EP 1520569.

The inventors have found that this problem can be ameliorated by use of elastic laminate portion (s) that, at least on the surface that faces the body in use, have wrinkles of (on average) a very small z-dimensions (height), and that have a very selected wrinkle density (wrinkles per cm). They found that such elastic laminate portions leave at the most very minor, shallow pressure marks (wrinkle indentations) on the skin, that disappear very quickly and that are (perceived as) less troublesome. Furthermore, if such minor pressure marks are still formed, they are believed to create less of a risk of skin irritation. They also found that it is beneficial that the elastic laminate portions (s) are very thin, at least in the free edge area.

Thus, absorbent articles are provided that still maintain an excellent elastic profile and performance and that at the same type have a highly reduced, or no pressure mark problem and that are more comfortable in use.

### SUMMARY OF THE INVENTION

The invention relates to a disposable absorbent article comprising a topsheet (20) having an opening and one or more longitudinally extending elastic laminate portions (10), formed by at least an elastic material (12) attached to the topsheet (20), said elastic laminate portion (s) (10) extending along at least part of said opening, said laminate portion (s) (10) comprising in at least relaxed and partially contracted state a body-facing surface with a multitude of wrinkles, and whereby said elastic laminate portion (10) has an absolute contracted length L_{c} and a fully stretched absolute length Lₛ,
whereby at a partial elongation of the elastic laminate portion (10) of ε = 0.5:
a) said wrinkles have an average wrinkle height H_{w} of less than 600 micrometers; and
b) said elastic laminate portion (10) has an average wrinkle density D_{w} between 5 and 10 wrinkles per cm, preferably between 5 and 9 wrinkles per cm, or in certain embodiments between 5 and 7.5 or even 7 wrinkles per cm.

Said elastic laminate portion (10) has in one embodiment an average caliper (at 0.33 psi (2,28kPa) and at an elongation ε = 0.5) up to 1.3 mm, typically up to 1.0 mm.

The article is preferably a diaper, e.g. an adult incontinence garment, baby or infant diaper or training pants.

Furthermore, the inventors have found that pressure marks may result in skin irritation if the pressure marks are caused by hydrophilic elastic material that may be wet in use, because the wetness can increase the skin irritation. Thus, the inventors have found that it is beneficial to ensure that such the nonwoven material, used in the laminate portions, are hydrophobic.

It may be preferred that the topsheet (20) itself is not elastically stretchable in y-direction.

The opening may be an elongated slit opening.

### BRIEF DESCRIPTION OF THE DRAWINGS

Figure 1 shows a perspective view of an exemplary diaper of the present invention
Figure 2 shows a topview of an exemplary diaper of the invention
Figure 3 shows a perspective view of section of a topsheet (20) with an elastic laminate portion (10), as shown in Figure 1.
Figure 4 shows the measurement results, obtained with the Primos method, using the Primos equipment and software, of a section of the elastic laminate portion (10), showing the wrinkle height and wrinkle width (and the there from derived density).
Figure 5 shows an exemplary attachment (adhesive (14)) pattern with attachment areas (14) that may be used to attach the elastic material to the topsheet material or other material comprised in the elastic laminate portion, as described herein.
Figure 6 shows an alternative attachment (adhesive (14)) pattern with attachment areas (14) that may be used to attach the elastic material to the topsheet material or other material comprised in the elastic laminate portion, as described herein.

### DETAILED DESCRIPTION

"Absorbent article" refers to wearable devices, which absorb and/ or contain liquid, and more specifically, refers to devices, which are placed against or in proximity to the body of the wearer to absorb and contain the various exudates discharged from the body. As used herein, the term "diaper" refers to an absorbent article generally worn by infants and incontinent people about the lower torso.

"y-direction" elongation or stretch as used herein means that the topsheet (20) or laminate portion (10) has as a whole an elongation or stretch in an average direction, that is herein referred to as "y-direction'. This may be a direction within 45 degrees of the longitudinal axis or line parallel thereto of the topsheet (20) or elastic laminate portion (10).

"Absolute fully stretched length" is the length of the topsheet (20) or elastic laminate portion (10) when said topsheet (20) or portion (10) is stretched in y-direction to its maximum length, as set out herein.

"Absolute contracted length" is the length of the topsheet (20) or elastic laminate portion (10), when no stretching force is applied to it, e.g. when it is in relaxed state, flat on a surface.

The "z-direction" is perpendicular to the y-direction and substantially in the direction of the wrinkle height in the elastic laminate portion (10), and is herein also referred to as the height or thickness dimension.

The "x-direction" is perpendicular to both the x- and y-directions.

As used herein, "elastic" means, that the item is extendible or stretchable by application of a force in a certain direction and returns to at least 80% of its original length but to less than 150% of its original length in that direction, and typically to about its original size, after the stretching force is released.

As used herein, "along" means at least partially parallel and in close proximity or even in contact with.

As used herein, the opening in the topsheet (20) is an area completely circumscribed by the topsheet (20), but where the topsheet (20) material is not present, and which is large enough to receive fecal material, typically being at least 2 cm long or wide, or having a surface area of at least 2 cm².

As used herein, the term "void space" is a cavity in the article present in at least the relaxed state, which serves to accept and contain bodily exudates such as fecal material, typically being at least 5 cm³ in relaxed state.

As used herein "attached" includes "directly attached" and "indirectly attached", e.g. via attaching "a" to "b" by attaching "a" to "c" and "c" to "b".

Each embodiment defined by certain properties or dimension for which a value is defined herein is to be understood to include embodiments with functional equivalent properties or dimensions, e.g. a dimension of 0.5 cm has to be understood as meaning "about 0.5 cm".

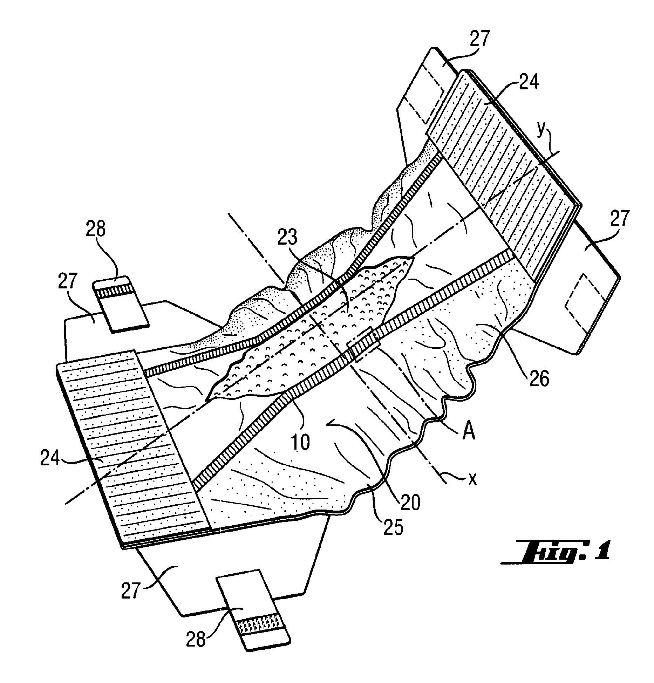

The disposable absorbent article of the invention may be a sanitary napkin, panty- liner, or a diaper, i.e. an adult incontinence garment or infant diaper (as shown in the Figures 1 to 5) or training or pull-up pants. The article comprises the topsheet (20) with an elastic laminate portion (10), described herein, and additional components, to have typically at least a backsheet (21), absorbent core (23) and a core cover sheet or topsheet (20).

The absorbent article of the invention comprises at least a topsheet (20) comprising at least one elastic laminate portion (10), formed from at least an elastic material (12) and a part of the topsheet material, e.g. the supporting topsheet material (13), that itself is typically not elastically stretchable, said elastic laminate portion (10) having at least y-directional stretch (elongation), or only y-directional stretch, as shown in the Figures. The article may also comprise other components that comprise such an elastic laminate portion(s) (10) such as leg cuffs (24) or barrier cuffs (26) or a waist band (s) (25).

The longest dimension or length of the topsheet (20) and of the elastic laminate portion (s) (10) are typically parallel to the y-axis of the topsheet (20) and of the article and this is typically substantially parallel to the average direction of stretch of the elastic laminate portion (10) and topsheet (20). If for example the leg cuffs (25) or barrier cuffs (26) comprise or consist of said elastic laminate portion (10) as well, then the y-direction of the leg cuffs (25) or barrier cuffs (26) and said laminate portion(s) (10) are typically substantially parallel or on average parallel to the y-direction and y-axis of the article.

Said topsheet (20) may comprise more than one of such laminate portions (10), which each may or may not be identical. For example, the topheet may comprise at least two separate, distinct elastic laminate portions (10), each of which is positioned along at least a part of the opening, e.g. such that at least one laminate portion (10) is positioned and extends along each longitudinal edge of the opening , or part thereof, as described herein after.

The topsheet (20) may consist of a (supporting) topsheet material and the elastic material (s) (12) or it may comprise other components, such as for example attachment means, attachment sheets, coverstrips (13) etc.

The elastic laminate portion (10) may be formed from the topsheet material (13) or part thereof, an elastic material (12) and optionally additional components, such as adhesive (14) or an attachment sheet positioned between the topsheet (20) and the elastic material (12), and/ or an additional cover-strip (13) that covers the opposite side of the elastic material, e.g. so that the elastic material may be sandwiched between two layers of topsheet materials, or between an attachment sheet and topsheet material(s), and/ or a cover-strip (13).

A preferred disposable absorbent articles of the invention is a diaper, including adult incontinent garments, pull-on or training diapers and baby or infant diapers with fasteners, as shown in the Figures, that may comprise an absorbent core (23); a liquid pervious core coversheet, positioned under the topsheet (20), on the absorbent core (23); a liquid impervious backsheet (21); optionally (elastic) side panels (27), (elastic) leg cuffs (25), (elastic) barrier cuffs (26), (elastic) waist feature (24), and a fastening system (28). The article as shown in Figure 1 has a first waist region, a second waist region, opposed to the first waist region and a crotch region located between the first waist region and the second waist region, each being about 1/3 of the length of the article.

In preferred embodiments, the backsheet (21) is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials which permit vapors to escape from the article while still preventing exudates from passing through the backsheet. Exemplary breathable materials may include materials such as woven webs, nonwoven webs, composite materials such as film-coated nonwoven webs, microporous films.

The core coversheet is preferably compliant, soft-feeling, and non-irritating to the wearer's skin. Further, at least a portion of the core cover sheet is liquid pervious, permitting liquids to be absorbed by the absorbent core (23) underneath. A suitable core cover sheet may be manufactured from a wide range of materials, such as porous foams, reticulated foams, apertured plastic films, or woven or nonwoven materials of natural fibers (e.g., wood or cotton fibers), synthetic fibers (e.g. polyester or polypropylene fibers), or a combination of natural and synthetic fibers. If the core cover sheet includes fibers, the fibers may be spunbond, carded, wet-laid, meltblown, hydroentangled, or otherwise processed as is known in the art.

Any portion of the core coversheet or the topsheet (20) described herein may be coated with a lotion as is known in the art. Examples of suitable lotions include those described in U.S. Pat. Nos. 5,607,760 entitled "Disposable Absorbent Article Having A Lotioned Topsheet Containing an Emollient and a Polyol Polyester Immobilizing Agent" issued to Roe on March 4, 1997. The lotion may function alone or in combination with another agent as the hydrophobizing treatment described above. The core coversheet and/ topsheet (20) may also include or be treated with antibacterial agents, some examples of which are disclosed in PCT Publication No. WO 95/24173 entitled "Absorbent Articles Containing Antibacterial Agents in the Topsheet For Odor Control" which was published on September 14, 1995 in the name of Theresa Johnson.

The absorbent core (23) may comprise any absorbent material which is generally compressible, conformable, non-irritating to the wearer's skin, and capable of absorbing and retaining liquids such as urine and other certain body exudates. The absorbent core may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt, and preferably at least superabsorbent polymers or absorbent gelling materials; or any other known absorbent material or combinations of materials. Preferred may be that the absorbent core comprises at least 80% by weight (based on the total content of material in the core, excluding the core wrap or cover sheet) of superabsorbent polymer or so-called absorbent gelling material.

The article may also include a fastening system (28) that maintains the first waist region and the second waist region in a configuration so as to provide lateral tensions about the circumference of the article to hold it on the wearer. The fastening system (28) preferably comprises a surface fastener such as tape tabs, hook and loop fastening components and/or hermaphroditic fastening components; although any other known fastening means are generally acceptable. In alternative embodiments, opposing sides of the article may be seamed or welded to form a pant. This allows the diaper to be used as a pull-on type diaper or training pant. Training pants are placed in position on the wearer by inserting the wearer's legs into the leg openings and sliding the training pant into position about the wearer's lower torso.

The article may also comprise side panels (27) that are preferably elastic or non-elastically extensible to provide a more comfortable and contouring fit by initially conformably fitting the article to the wearer and sustaining this fit throughout the time of wear well past when it has been loaded with exudates since the elasticized or extensible side panels allow the sides of the article to expand and contract.

The article may include a pair of longitudinally extending, transversely spaced apart barrier cuffs (26), and/ or longitudinally extending, transversely spaced apart leg cuffs (25), which provide improved containment of liquids and other body exudates. Said leg cuffs and/ or barrier cuffs may also comprise the elastic laminate portion (10) as described herein below

### Topsheet (20) with the elastic laminate portion(s) (10)

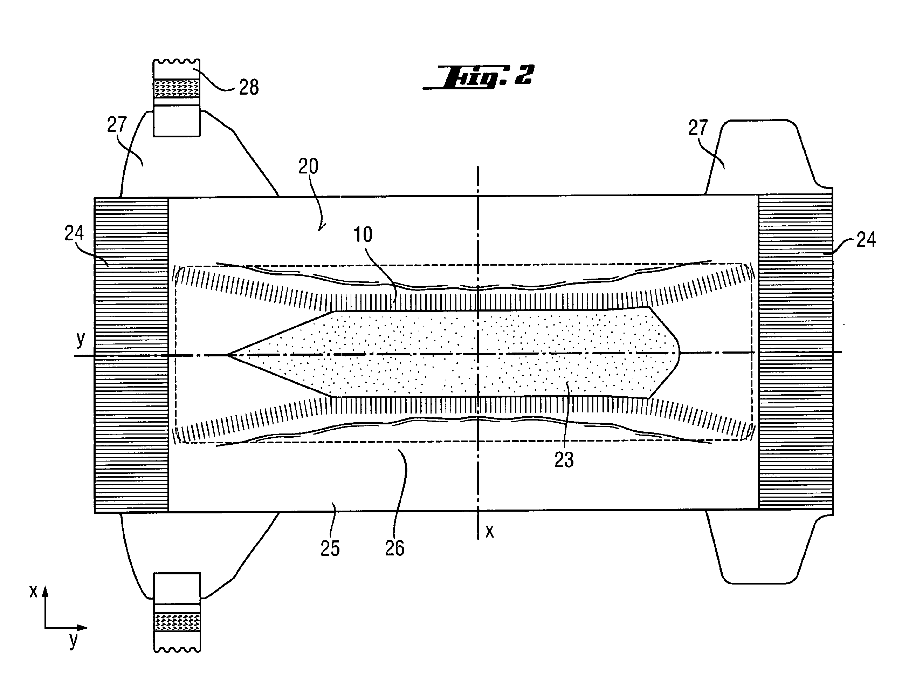

The topsheet (20) comprises at least one elastic laminate portion (10), but in one embodiment it preferably comprises at least two separate elastic laminate portions (10), as also shown in the Figures 1 and 2.

Each laminate portion (10) has in relaxed/ contracted state and in partially stretched state, including at an elongation ε of 0.5 as described herein below, at least one surface with wrinkles that face the user's body and may be in contact with the skin in use. The elastic laminate portion (10) may comprise two or more (preferably non-elastic) sheets of material that are attached to either surface of an elastic material, and the laminate portion than typically contains wrinkles on either surface of the elastic laminate portion.

The properties of said wrinkles described herein applies at least to the wrinkles of the body-facing surface of the elastic laminate portion (10), but may apply to both surfaces of said laminate (10).

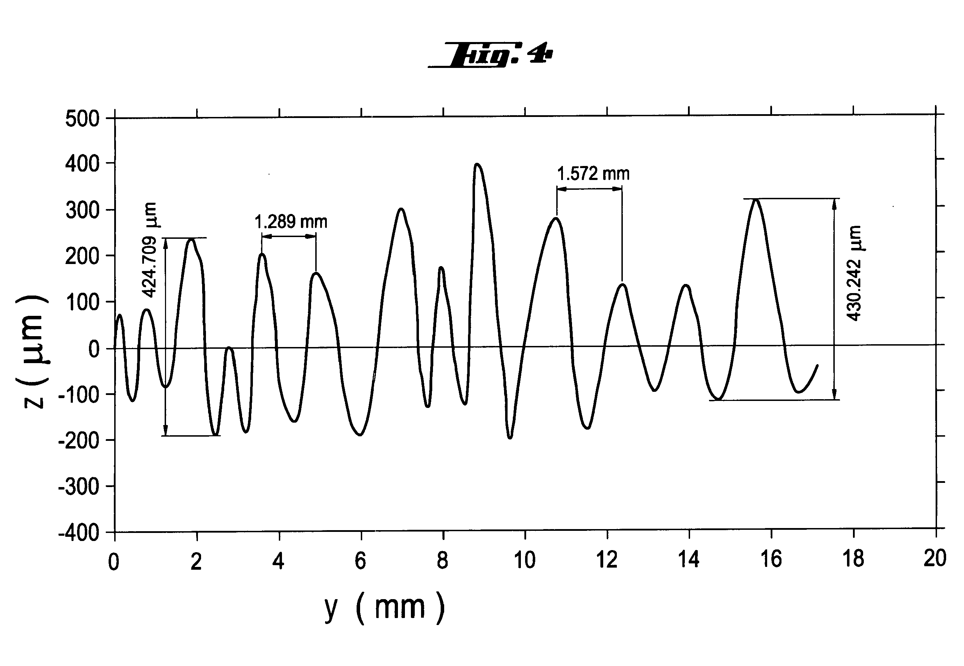

At an elongation ε of 0.5, whereby ε = (Lₓ- L_{c}) / L_{c}), said wrinkles are of an average wrinkle height (as measured by the "Primos" method set out below, using PRIMOS equipment) of less than 600 microns, and typically less than 600 but more than 200 microns, or between 550 microns and 300 microns, or up to 530, or even up to 500 microns. An exemplary Primos measurement graph of a section of an elastic laminate portion (10) as submitted by the test method herein is shown in Figure 4.

At an elongation ε of 0.5, the elastic laminate portions (10) herein may have an average wrinkle density (wrinkles per cm) of from 5 to 10 wrinkles per cm or even from 6 to 10, or even 7 to 10, or possibly only up to 9 wrinkles per cm, as measured with the Primos method set out below. The elastic laminate portions (10) of the invention have an average maximum elongation εₘₐₓ, (being (Lₛ- L_{c})/ L_{c} whereby Lₛ is the fully stretched length of the elastic laminate portion), of at least 0.8 or even more preferably at least 1.0 or even more preferably at least 1.2, or it may be at least 1.4. These values can be obtained by the method set out in the method section below. It should be understood that the topsheet (20) may comprise areas (typically of between 0.5 and 2 cm, or 0.8 and 1.5 cm) where an elastic material is attached to the topsheet material without providing in that area any elongation of at least 0.5, and there are then possibly not even any wrinkles present. It should be understood for the purpose of the invention that such areas where elastic material is present but that have an elongation of less than 0.5 are not considered part of the elastic laminate portion (10) herein. Such areas may herein be referred to as "attachment portions".

In order to determine and obtain the elongation ε = 0.5 of the elastic laminate portion (10), the elastic laminate portion's (10) absolute contracted length L_{c}, is first determined as follows.

The topsheet (20) with the elastic laminate portion (10) is removed from the absorbent article, or if possible the elastic laminate portion (10) is removed from the article, either way such that the wrinkle profile and elastic profile (i.e. of the upward facing surface that in use is facing the body of the user) is not changed.

The topsheet (20) or elastic laminate portion (10) is placed as flat as possible on a surface, without applying any elongating or stretching force to it. Then, the absolute contracted length of the elastic laminate portion (10) of the topsheet (20) is measured. This is herein referred to as the absolute contracted length of the laminate L_{c}.

Then, the length of the laminate portion (10) at ε = 0.5 can be calculated, this being 1.5 Lc (based on : ε = (Lₓ- L_{c}) / L_{c}).

Subsequently, the laminate portion (10) can be stretched by the method described herein below to obtain this elongation of 0.5 at 1.5 L_{c}. Then, the wrinkle heights, average wrinkle height and deviations, wrinkle width, distance between wrinkles, average wrinkle density and deviation thereof can be calculated by use of the Primos method, using PRIMOS equipment, as described below.

Typically, the elastic laminate portion (10) has wrinkles of a relatively or substantially uniform wrinkle height (distribution), at least on the body-facing surface of the elastic laminate portion (10). For example, less than 10% or preferably less than 5% of the wrinkles are 800 microns or more, preferably less than 10% or even less than 5% of the wrinkles are 700 or more, or even 650 microns or more; and it may even be possible that 95% or more, or even all wrinkles (about 100%) have a height of less than 600 microns.

Furthermore, it may be preferred that the wrinkle density is substantially uniform throughout an elastic laminate portion e.g. that in no section of 2 cm (in length direction, along the laminate portion) the wrinkle density is more than 12 and that in no section of 2 cm (in length direction, along the laminate portion) the wrinkle density is less than 3, or less than 4. (Preferred may be that in each 2 cm section of a laminate portion (10) the wrinkle density is between 5 and 10, or any of the preferred values described above.

The width of the elastic laminate portions (10) will vary, typically depending on the exact dimensions of the topsheet (20) and/ or of the article.

For example, for size 4 diapers the elastic laminate portion (10) in a topsheet (20) herein may, in fully stretched state, have an average width of about 3 mm to 50 mm, preferably 3 mm to 40 mm, or even more preferably 3mm, or even 5 mm to 20 mm.

The elastic materials (12) used herein are typically very thin, typically having a thickness or caliper (e.g. gauge) of typically up to about 200 microns, or even up to 150 microns or even up to 110 microns, or up to 100 microns and they may need to be at least 20 microns, more preferably at least 40 microns, or even at least 60 microns, as defined herein. Highly preferred materials have a thickness of about 70 to 100 microns.

A suitable elastic material is for example 2L-89, available from Fulflex, (Limerick, Ireland).

The topsheet (20) herein has at least one opening, or only one such opening, and said opening(s) may be in the form of a (single) slit opening, as shown in Figures 1 and 2. The opening is present in the crotch region and preferably in part of the front waist region of the topsheet (20) (in use towards the front of the user) and in part of the back waist region of the topsheet (20). Preferably, the topsheet (20) has a slit opening, which has a longitudinal dimension (length) substantially parallel to the longitudinal axis of the topsheet (20) and of the diaper. Preferred is that (in stretched state) the opening (or openings) of the topsheet (20) is (are) configured such that from 20% to 40%, or more preferably from 20% to 30% of the length of the opening (or total length of the openings) extends from the transverse axis of the topsheet (20) towards the front edge of the topsheet (20), and the remaining percentage extends towards the back edge of the topsheet (20).

The dimensions and exact shape of the opening(s) may vary, depending on the size of the topsheet (20) and/ or the absorbent article. For example, in a preferred embodiment the opening is in the form of a slit opening with substantially parallel longitudinal side edges, which are connected in the front and/ or back by V-shaped or U-shaped edges. In a preferred embodiment, the front V-shaped edges have a larger angle than the back V-shaped edges. Preferably, the front V-shaped edges have an angle of 20° to 140°, preferably from 45° to 65 °, most preferably from 55° to 60°, as described herein after and can be seen from Figures 1 and 2.

The maximum length of the slit opening may be for example 40% to 90% or more preferably 50% to 80%, or even more preferably about 60% to 70%, of the total fully stretched length Ls of the absorbent article.

Preferred may be, in particular for size 4 diapers with a maximum topsheet (20) length of between 45 cm and 55 cm, typically between 48 cm and 52 cm, that the length of the single slit opening, when the diaper is in fully stretched state, is from 20 cm to 40 cm, or even from 25 cm to 35 cm, or even from 28 cm to 32 cm.

The average width of the opening herein, in fully stretched state, is preferably from 5% to 30%, or more preferably 10% to 25%, of the average width of the topsheet (20) (including opening width), or for example for a size 4 diaper, 15 mm to 60 mm, more preferably from 20 mm to 40mm.

The topsheet (20) comprises in one embodiment herein at least two elastic laminate portions (10), to form typically a pair of opposing, preferably at least partially parallel and/ or at least partially longitudinally extending elastic laminate portions (10), (elasticated areas), or preferably mirror image elastic laminate portions (10), such as described in copending application EP-A-1201212, and shown in Figures 1 and 2.

The elastic laminate portion (10) extends preferably along the longitudinal side edges of the opening(s) towards or completely to the front and/ or back transverse edge of the topsheet (20). Thus, the elastic laminate portions (10) are preferably longer than the opening. Preferred is that the elastic laminate portions (10) are positioned over the full length of the topsheet (20), or at least the part of the topsheet (20) which in use is intended to receive body exudates, typically the topsheet (20) minus the parts thereof which form (part of) the waist bands, if present.

The elastic laminate portion (10) is preferably shaped such that it has a centre portion that is substantially parallel to the centre portion of the opposing elastic laminate portion (10).

Each of the two centre portions has a length, which is preferably 30% to 70% of the total fully stretched length Ls of a corresponding elastic laminate portion (10), and preferably about 40% to 80% of the maximum length of the opening; hereby, it is preferred that the total length of the elastic laminate portion (10) is about 70% to 90%, or preferably from about 80% to 90% or preferably about 85% of maximum length of the topsheet (20).

Preferred is that the front end portions of two opposing elastic laminate portions (10) bend away from one another (in the plane of the topsheet (20)), so that the distance between the end edges of the opposing front end portions of two opposing elastic laminate portions (10) is larger that the distance between the centre portions of two opposing elastic laminate portions (10), and equally, the distance between the end edges of the opposing back end portions of two opposing elastic laminate portions (10) is larger that the distance between the centre portions of two elastic laminate portions (10).

The elastic laminate portion (10) is typically angled, as described herein, whereby it has a front and back angle with a longitudinal line through the centre straight part (or centre portion) of the elastic laminate portion (10) and parallel to the longitudinal axis of the topsheet (20), of between 10° and 40°, or preferably between 17° to 35°, or even more preferably between 20° and 35°. Such pair of opposing, angled elastic laminate portions (10) are herein referred to, as having an X-shape, and a preferred X-shape is exemplified in Figure 2.

The free longitudinal edge of the elastic laminate portion (10) or the laminate portion (10) as a whole may have an average caliper (at a pressure of 0.33 psi (2,28 kPa) and an elongation of 0.5) of 1.3 mm or less, or1.1 mm or less, or 1.0 mm or less, or 0.95 mm (950 microns) or less, and it may be more than 0.4 mm (400 microns) or even for example more than 0.6 mm (600 microns), as measured with the method set out herein below.

Hereto, the elastic laminate portion (10) may comprise one or two (laminated) layers that each have a basis weight of 20g/m² or less, preferably one or two (laminated) layers of a basis weight of 17 g/m² or less; provided that if two (laminated) layers are present, their combined basis weight may preferably be 34 g/m² or less.

Typically, at least one topsheet material (13) forms part of the laminate portion (10), and hence, preferred is that at least one layer of the elastic laminate portion (10) is part of the topsheet material (13) or the topsheet (20) as a whole.

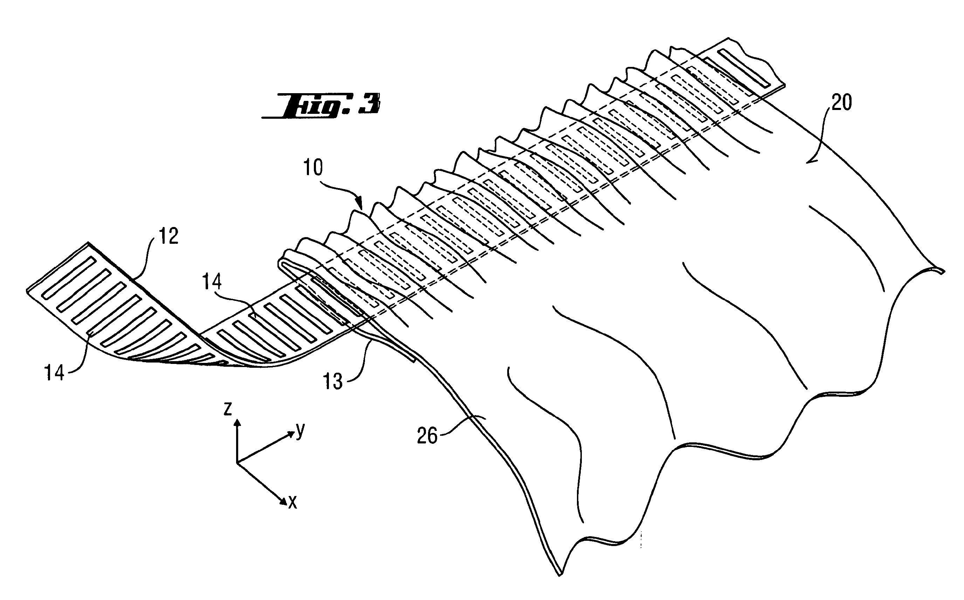

The topsheet (20) may also be made of two (laminated) layers, with therein between, in certain area(s), the elastic material (e.g. a so-called sandwich structure), as for example shown in Figure 3. Then, for example, the topsheet (20) may be made of a single (laminated) layer of for example a basis weight of 20 g/m² or less, or one or two (laminated) layers of a basis weight of 17 g/ m² or less.

The topsheet (20) as a whole may typically have an average caliper that is the same or less than the caliper of the laminate portion (10).

The topsheet (20) and the elastic laminate portion (10) herein may comprise any sheet material suitable to be laminated to an elastic material (12). Preferably, the sheet material of the topsheet (20) and laminate portion (s) (10) is not itself elastically stretchable in y-direction.

The topsheet (20) is preferably air permeable. In certain executions herein, it may be preferably that it has high barrier properties. The topsheet (20) and the elastic laminate portion (10) is preferably hydrophobic and/ or urine-impermeable.

Preferred topsheets (20) and elastic laminated portion(s) (10) thereof comprise woven and non-woven materials of natural fibers (e.g., wood or cotton fibers) and/ or synthetic fibers. They comprise preferably thermoplastic polymer fibers, preferably selected from the group comprising: polyolefins, polyesters, polyurethanes, and polyamides, most preferably the thermoplastic polymer being a polyolefin, most preferably being polypropylene or polyethylene. The fibers may be spun bond, carded, wet-laid, melt blown, and/ or hydro entangled, and/or otherwise processed as is known in the art. Preferred is that the topsheet (20) and laminate portion (10) thereof comprises one or more nonwoven material sheets that is itself a laminate of layers of meltbown and carded or spunbond material. For example, a sheet or layer of the topsheet may be a laminate of at least two layers, one of which at least is a meltblown (M) layer and one of which is at least a spunbond (S) or a carded (C) layer.

Preferred executions are SM SMS, SMMS, SSMS, SSMSS, SSMMS, CM or CMC non-wovens laminates.

Most preferably, said non-woven webs are formed from polyethylene, polypropylene and/ or polybutylene polymer fibers, or (a mixture of) fibers of a copolymers of polyethylene, polypropylene and/ or polybutylene; most preferred are polypropylene polymer fibers.

It may also be preferred that the topsheet (20) comprises ingredients, which reduce friction between the wearer's skin and the topsheet (20), or in particular between the skin and the elastic laminate portion (10). Hereto, the laminate portion (10) or topsheet (20), may for example comprise a lotion, a fine powder, such as talcum powder, or wax.

The topsheet (20) or laminate portion (10) may be treated with an agent to reduce its surface energy. For example useful agent include fluorocarbons as described in U.S. Patent 5,876,753, issued to Timmons et al. on March 2, 1999; U.S. Patent 5,888,591 issued to Gleason et al. on March 30, 1999; U.S. Patent 6,045,877 issued to Gleason et al. on April 4, 2000. Other agents include silicone. Useful methods for applying the agent to the material, without reducing the air permeability, can be found in U.S. Patent 5,322,729 and PCT Publication WO 96/03501. Preferred agents may be selected from the group comprising fluorocarbons, siloxanes, polysiloxanes, preferably including fluorinated monomers and fluorinated polymers, including hexafluoroethylene, hexafluoropropylene and vinyl fluoride and vinylidene fluoride, fluoroacrylate and fluoromethacrylate. Highly preferred is that the topsheet (20) is provided with poly (tetra) fluoroethylene, fluorinated ethylene-propylene copolymers and/ or fluorinated ethylene-tetrafluoroethylene copolymers.

The topsheet (20) with the elastic laminate portion (10) and/ or the laminate portion (s) (10) herein are such they typically have one of the following elastic profiles:
a) 1.5Lt by a first load force of less than 1.1N or even less than 0.6N, 3.0Lt by a first load force of less than 2.1N or even 1.1N and 4.5Lt by a first load force of less than 3.0N or even less than 1.5N and a second unload force at 4.5Lt of more than 0.9N, a second unload force at 3.0Lt of more than 0.5N and a second unload force at 1.5Lt of more than 0.1N.
   (Said elastic profile obtainable by the method set out in co-pending application EP1201212-A, whereby Lt is the contracted length of the topsheet (20) or elastic laminate portion (10), which ever applicable, herein referred to as L_{c}).
   or :
b) 0.25Lₛ by a first load force of less than 0.6 N, 0.55Lₛ by a first load force of less than 5N or even less than 3.5 N and 0.8Lₛ by a first load force of less than 10.0N or even less than 7.0N and a second unload force at 0.55Lₛ of more than 0.4N, and a second unload force at 0.80Lₛ of more than 1.4N, or even more than 2.0N.
   (Said elastic profile obtainable by the method set out in co-pending application EP1201212-A, whereby Lₛ is as specified herein, being the fully stretched length of the elastic laminate portion (10) or topsheet (20), which ever is applicable.)

Preferably, the topsheet (20) and/ or elastic laminate portion (10) has a force profile such that it has a first load force at 200% elongation of 1.6 N or less, and a second unload force at 200% elongation of 0.5 N or more.

The elastic material (12) may be attached to one or more of the supporting sheet(s) or layers or nonwoven(s) that form the elastic laminate portion (10) or typically the topsheet, by any method, including adhesive bonding and ultrasonic bonding, but preferred may be the use of adhesive (14). The adhesive (14) may be applied such that the required wrinkle density and wrinkle heights are achieved, as also claimed herein.

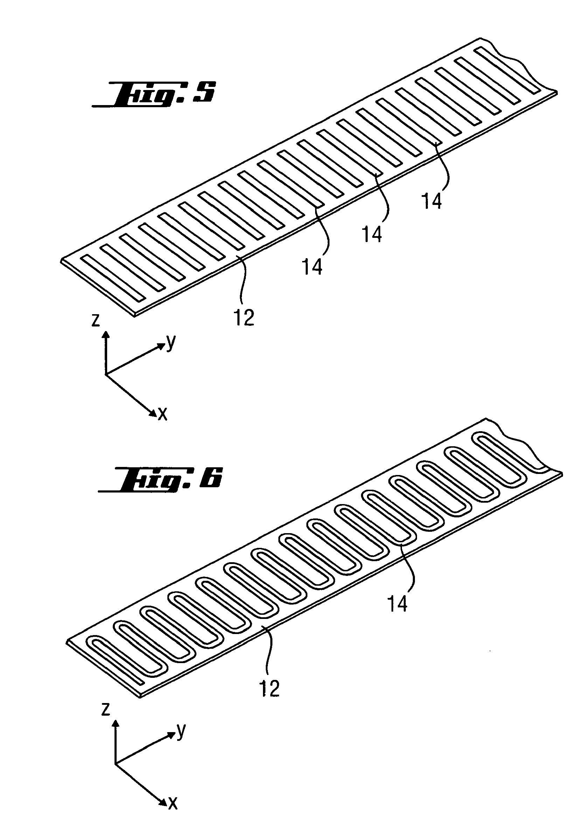

Preferred is that the elastic material (12) is attached to the topsheet and/ or to other material comprised in the elastic laminate portion by use of a specific pattern that is suitable to obtain the wrinkle heights and densities referred to herein, said pattern having providing attachment areas (14) and non attachment areas therein between, as for example shown in Figures 5 and 6.

Preferred may be that the elastic laminate portion (10) has, at fully stretched absolute state with length Lₛ, substantially transverse attachment areas (14) such that the average distance between neighboring transverse parts of the attachment area(s) (14) is from 0.3 mm to 2.5 or to 2.0 mm, or preferably from 0.6 to 1.2 mm. Preferred may be that the average width (substantially in the direction of stretch), of the transverse parts (in the elastic laminate portion (10) at said fully stretched state), is from 0.2 mm to 1.2 mm, or preferably from 0.3 mm to 0.8 mm. or even 0.5 or less.

For example, the attachment means may include a uniform continuous layer of adhesive (14), a patterned layer of adhesive (14), or an array of separate lines, spirals, "omega" shaped line(s),or spots of adhesive (14). The adhesive (14) may for example be applied in an intermittent stripe pattern, preferably straight stripes that are positioned in (substantially) transverse direction along the elastic laminate portion, e.g. substantially perpendicular to the longitudinal direction of the elastic laminate portion (10), as shown in Figure 5. Preferred may be that the average distance between neighboring adhesive (14) stripes (attachment area (14), as also shown in Figure 5) is from 0.3 mm to 2.5 or even to 2.0 mm, or preferably to 1.5 mm, or even from 0.6 to 1.2 mm. Preferred may be that the stripes are have on average a width (in y-direction of stretch) of less than 1.2 mm, preferably 0.3 to 0.8 mm, or even 0.5mm or less.

Preferred may be that the ratio of the average width of the stripes to the average distance between neighboring stripes (ratio R_{adhesive}) is from about 4:10 to 8:10 or 6:10, or for example about 1:2

The adhesive (14) may also be applied in a so-called omega pattern, as shown in Figure 6, whereby each omega shaped part has a "loop", with a upwards "leg" and a downside "leg", departing from and arriving to an average "base-line". Hereby, the omega line itself may have an average width as described above for the width of the attachment areas (14) and the distance between the neighboring "legs of a loop or neighboring loops" may be as described above for the distance between the attachment areas (14) ( and then each "leg" is considered an attachment area (14); or the distance between neighboring "loops" may be as described herein above for the distance between neighboring attachment areas (14) and the average width of a loop may be as described above for the average width of an attachment area (14) (and then the loop as a whole is an attachment area (14)). In either case, the R_{adhaive} may also be as above.

### Test methods:

### Method to stretch the elastic laminate portion (10) to an elongation of ε= 0.5;

### Method to determine its fully stretched length Lₛ and εₘₐₓ

The elastic laminate portion (10) may be straight, curved, or it may comprise several straight parts that are joined under one or more angles with one another, as can been seen in Figures 1 and 2, or it may have a combination of such configurations. This is herein referred to, respectively, as "straight", "curved" or "angled" elastic laminate portion (10), respectively, or for example, "curved and angled" elastic laminate portion (10) etc.

In each case below, a sample (e.g. topsheet. or preferably an elastic lamainate portion thereof, if this can be isolated as such) is obtained from an absorbent article that has been conditioned for 24 hours at 50% humidity and 23 C.

### 1) When the elastic laminate portion (10) is straight:

The elastic laminate portion (10) or topsheet as a whole is obtained and put on a flat surface as described above to measure the contracted length of the elastic laminate portion (10) L_{c}.

The elastic laminate portion is subsequently elongated to 1.5 L_{c} (equals ε =0.5) or to its fully stretched length Lₛ, to determine εₘₐₓ as follows.

The sample (the topsheet with the elastic laminate portion or the elastic laminate portion thereof) is left for 24 hours at 25°C and 50% humidity, prior to the elongation/ stretching step below, which is subsequently performed under the same conditions.

Measurement of lengths of the samples can be done with a micrometer screw.

The sample to be tested is placed length-wise (in the direction of stretch) between two tweezers or, if the width of the sample is more than 1cm, between two clamps of a width of 1 cm, one on each end, such that contact area of the tweezers/ clamp and the sample is at the most 1 mm for clamps and 0.5mm for tweezers in the direction of stretch (length). The exact distance between the start of one clamp or tweezers to the beginning of the other clamp or tweezers is measured. This is the contracted length of the sample, e.g. of the laminate portion.

For straight samples, the clamps or tweezers are moved in the y-direction of the length of the straight samples, such that the length direction is the direction of the elongation force.

The sample may thus be stretched to its maximum elongation (e.g. when the supporting topsheet material (13) reaches its maximum length) and the length of the sample and the distance between the clamps is measured, and the elongation εₘₐₓ is calculated.

Alternatively, the sample is stretched to ε = 0.5, in order to submit the thus stretched sample to the Primos method set out below.

### 2) When the elastic laminate portion (10) is "angled":

The elastic laminate portion (10) is divided by marking with a fme marker pen into straight parts (i.e. between the angles), for example in 3 straight parts. The sample is prepared and conditioned as described above.

Subsequently, each straight part is elongated separately by the method set out above for straight elastic laminate portions (10) to either ε = 0.5 or εₘₐₓ, e.g. when the sample comprises two angles and 3 straight parts, 3 force lines are determined and the sample is stretched 3 steps.

### 3) When the elastic laminate portion (10) is curved:

The curved elastic laminate portion (10) is divided with a fine marker pen into sections of 2 cm absolute length and possibly one remaining section of a smaller length. The sample is prepared and conditioned as described above.

Prior to elongation, the force line of each section of 2 cm (or one section of less than 2 cm) is determined as follows. Each section has two transverse edge lines that are 2 cm apart, and each transverse edge line has a centre point. A line can be drawn through said two points of said two transverse edge lines. This will be the "y-direction line" or force line along which the force will be applied to elongate said section. This will be done for each section.

Subsequently, each section is elongated separately by the manner set out above for straight elastic laminate portions (10), but by separately elongating each section along its own force line, to either ε = 0.5 or its maximum elongation εₘₐₓ.

After stretching all sections, a fully stretched absolute length can be measured for each section and for the elastic laminate portion (10), Lₛ and εₘₐₓ can be calculated.

### 4) Mixed elastic laminate portion (10)

If the elastic laminate portion (10) comprises a combination of curved, angled and/ or straight parts, then a combination of the above methods is applied accordingly.

### Primos method:

### Determination of the wrinkle heights and density, averages and deviations thereof

The following described the method to determine the wrinkle height and winkle density of the laminate portion (10) of the topsheet (20).

Each sample with the elongation of 0.5 as defmed and obtained by the method described herein, is examined by use of PRIMOS equipment and its data acquisition software, following the manufacture's instructions manual, using a 13x18mm lens.

If the elastic laminate portion (10) has an average width of more than 3 mm, then the measurement above is only done on the inner 70% of the width of the laminate portion, along its length.

The PRIMOS equipment will provide graphs per measured section of the sample , as shown in Figure 4, and it provides exact values per wrinkle, e.g. height, width, and it allows to calculate the average of wrinkles per cm, wrinkle height, deviations etc.

### Caliper measurement

The caliper and average caliper of an elastic laminate portion (10) that has an elongation of ε= 0.5, as described herein, can be obtained by use of a micrometer, such as the Frank 16303, obtainable from Twing Albert-Frank GmbH. The test is done at 23 °C, 50% humidity. The sample should be already conditioned to this humidity and temperature as set out above, since it has been conditioned for 24 hours under these conditioned, prior to stretching it to the required elongation of 0.5, needed to do this caliper test.. The equipment is calibrated prior to testing. The lowering speed of the pressure foot is set to be 3 mm/ sec and the dwelling time 2-5 sec.

The size (surface area) of the anvil is chosen depending on the size of the elastic laminate potion (10), and subsequently the weight on the pressure foot is chosen such that the required pressure of 0.33psi (2,28 kPa) is obtained.

For example, an anvil with a 40 mm diameter is used and a total weight of 295 grams (80 grams of the pressure foot plus an additional 115 grams) is applied to measure preferred elastic laminate portion(s) (10) herein.

To obtain the average caliper of the elastic laminate portion, the test is repeated on several portions of the elastic laminate portion, such that the areas pressed by the anvil per measurement do not overlap. Subsequently, an average can be calculated. Also the deviation in the caliper can be calculated.

## Claims

1. A disposable absorbent article comprising a topsheet (20) having an opening and one or more longitudinally extending elastic laminate portions (10), formed by at least an elastic material (12) attached to the topsheet (20), said elastic laminate portion (s) (10) extending along at least part of said opening, said laminate portion (s) (10) comprising in at least relaxed and partially contracted state a body-facing surface with a multitude of wrinkles, and whereby said elastic laminate portion (10) has an absolute contracted length L_{c} and a fully stretched absolute length Lₛ, **characterised in that**:
at a partial elongation of the elastic laminate portion (10) of ε = 0.5:
a) said wrinkles have an average wrinkle height H_{w} of less than 600 micrometers; and
b) said elastic laminate portion (10) has an average wrinkle density D_{w} between 5 and 10 wrinkles per cm.

2. An absorbent article as in claim 1 whereby said elastic laminate portion (10) has an average caliper (at 0.33 psi (2,28 kPa) and at an elongation ε = 0.5) from 0.4 mm to 1.1 mm, or even to 1 mm.

3. A disposable absorbent article as in claim 2, whereby laminate portion (10) comprises one or two nonwoven laminate sheets that have a basis weight of 22 g/ m² or less, preferably 20 g/m², 17 g/ m², or 13 g/m², preferably having one or two nonwoven laminate sheets with each a basis weight of 17 g/m² or less.

4. A disposable absorbent article as in any of claim 1 to 3, whereby said elastic material (12) is attached to the topsheet (20) by use of attachment areas (14), typically comprising an adhesive, said attachment areas (14) being in the form of applied an omega pattern with transverse attachment area loop parts, or in the form of an intermittent transverse stripe attachment areas (14), whereby at fully stretched state Ls, the average distance between neighboring transverse attachment areas or area parts (14) is between 0.3 to 2.2 mm, or between 0.6 and 1.2 mm.

5. A disposable absorbent article as in any claim 4, whereby the average width of the attachment areas (14) (in y-direction; at fully stretched state) is between 0.3 mm and 0.8 mm, preferably 0.5 mm or less, and/ or the ratio between said average width and said average distance is 4: 10 to 8:10 or 6:10, or preferably 1:2.

6. A disposable absorbent article as in any preceding claim, whereby at least the body-facing surface of said topsheet (20) and/ or said elastic laminate portion (10) is hydrophobic, preferably having a surface energy of less than 30 dynes/ cm, or even less than 27 dynes/cm.

7. A disposable absorbent article as in any preceding claim whereby the topsheet (20) has a force profile such that it has a first load force at 200% elongation of 1.6 N or less, and a second unload force at 200% elongation of 0.5 N or more.

8. A disposable absorbent article as in any preceding claim, which is a diaper or adult incontinence garment, whereby said opening is a elongated slit opening, and whereby the topsheet (20) comprises at least two elastic laminate portions (10), that are laterally opposing and extend along the longitudinal edges of the opening such that at least one elastic laminate portion (10) extends along each longitudinal edge of the opening towards or to the front transverse edge and/ or back transverse edge of the diaper or garment.

## Patentansprüche

1. Einweg-Absorptionsartikel, umfassend eine Oberschicht (20) mit einer Öffnung und einem oder mehreren längs verlaufenden elastischen Laminatabschnitten (10), die wenigstens durch ein elastisches Material (12) gebildet werden, das an der Oberschicht (20) befestigt ist, wobei der elastische Laminatabschnitt bzw.
die elastischen Laminatabschnitte (10) sich entlang wenigstens einem Teil der Öffnung erstreckt bzw. erstrecken, wobei der Laminatabschnitt bzw. die Laminatabschnitte (10) wenigstens im entspannten und im teilweise kontrahierten Zustand eine körperseitige Oberfläche mit einer Vielzahl von Falten umfasst bzw. umfassen und wobei der elastische Laminatabschnitt (10) eine absolute kontrahierte Länge Lₖ und eine vollständig gestreckte absolute Länge L_{g} besitzt, **dadurch gekennzeichnet, dass**:
bei einer teilweisen Dehnung des elastischen Laminatabschnitts (10) von ε
= 0,5:
a) die Falten eine durchschnittliche Faltenhöhe H_{F} von weniger als 600 Mikrometer besitzen; und
b) der elastische Laminatabschnitt (10) eine durchschnittliche Faltendichte D_{F} zwischen 5 und 10 Falten pro cm besitzt.

2. Absorptionsartikel nach Anspruch 1, wobei der elastische Laminatabschnitt (10) eine durchschnittliche Dicke (bei 2,28 kPa (0,33 psi) und bei einer Dehnung ε = 0,5) von 0,4 mm bis 1,1 mm oder sogar bis 1 mm besitzt.

3. Einweg-Absorptionsartikel nach Anspruch 2, wobei der Laminatabschnitt (10) eine oder zwei Vlieslaminatschichten umfasst, die ein Basisgewicht von 22 g/m² oder weniger, vorzugsweise 20 g/m², 17 g/m² oder 13 g/m² besitzen, vorzugsweise eine oder zwei Vlieslaminatschichten mit einem Basisgewicht von jeweils 17 g/m² oder weniger besitzend.

4. Einweg-Absorptionsartikel nach einem der Ansprüche 1 bis 3, wobei das elastische Material (12) an der Oberschicht (20) mittels Befestigungsflächen (14) befestigt ist, die üblicherweise ein Haftmittel umfassen, wobei die Befestigungsflächen (14) in Form eines Omega-Musters mit quer verlaufenden, schlingenförmigen Befestigungsflächenteilen oder in Form von intermittierenden, quer verlaufenden, streifenförmigen Befestigungsflächen (14) aufgebracht sind, wobei im vollständig gestreckten Zustand Lg der durchschnittliche Abstand zwischen benachbarten quer verlaufenden Befestigungsflächen oder Befestigungsflächenteilen (14) zwischen 0,3 und 2,2 mm oder zwischen 0,6 und 1,2 mm beträgt.

5. Einweg-Absorptionsartikel nach Anspruch 4, wobei die durchschnittliche Breite der Befestigungsflächen (14) (in y-Richtung; in vollständig gestrecktem Zustand) zwischen 0,3 mm und 0,8 mm beträgt, vorzugsweise 0,5 mm oder weniger, und/oder das Verhältnis zwischen der durchschnittlichen Breite und dem durchschnittlichen Abstand 4:10 bis 8:10 oder 6:10 beträgt, oder vorzugsweise 1:2.

6. Einweg-Absorptionsartikel nach einem der vorstehenden Ansprüche, wobei wenigstens die körperseitige Oberfläche der Oberschicht (20) und/oder des elastischen Laminatabschnitts (10) hydrophob ist und vorzugsweise eine Oberflächenenergie von weniger als 30 Dyn/cm oder sogar weniger als 27 Dyn/cm besitzt.

7. Einweg-Absorptionsartikel nach einem der vorstehenden Ansprüche, wobei die Oberschicht (20) ein derartiges Kraftprofil besitzt, dass sie eine erste Belastungskraft bei 200 % Dehnung von 1,6 N oder weniger und eine zweite Entlastungskraft bei 200 % Dehnung von 0,5 N oder mehr besitzt.

8. Einweg-Absorptionsartikel nach einem der vorstehenden Ansprüche, der eine Windel oder ein Inkontinenz-Kleidungsstück für Erwachsene ist, wobei die Öffnung eine längliche Schlitzöffnung ist und wobei die Oberschicht (20) mindestens zwei elastische Laminatabschnitte (10) umfasst, die sich seitlich gegenüberliegen und sich entlang den Längsrändern der Öffnung erstrecken, so dass sich mindestens ein elastischer Laminatabschnitt (10) entlang jedem Längsrand der Öffnung in Richtung auf den oder zum vorderen Querrand und/oder hinteren Querrand der Windel oder des Kleidungsstücks hin oder bis zum vorderen Querrand und/oder hinteren Querrand der Windel oder des Kleidungsstücks erstreckt.

## Revendications

1. Article absorbant jetable comprenant une feuille de dessus (20) ayant une ouverture et une ou plusieurs parties stratifiées élastiques s'étendant longitudinalement (10), formées par au moins un matériau élastique (12) fixé à la feuille de dessus (20), ladite ou lesdites partie(s) stratifiée(s) élastique(s) (10) s'étendant le long d'au moins une partie de ladite ouverture, ladite ou lesdites partie(s) stratifiée(s) élastique(s) (10) comprenant dans un état au moins relaxé et partiellement contracté une surface faisant face au corps avec une multitude de plis, et selon quoi ladite partie stratifiée élastique (10) a une longueur absolue contractée L_{c} et une longueur absolue complètement étirée Lₛ, **caractérisé en ce que** :
à un allongement partiel de la partie stratifiée élastique (10) de ε = 0,5 :
a) lesdits plis ont une hauteur moyenne de pli H_{w} de moins de 600 micromètres ; et
b) ladite partie stratifiée élastique (10) a une densité moyenne de plis D_{w} comprise entre 5 et 10 plis par cm.

2. Article absorbant selon la revendication 1, selon lequel ladite partie stratifiée élastique (10) a une épaisseur moyenne (à 2,28 kPa (0,33 psi) et à un allongement ε = 0,5) allant de 0,4 mm à 1,1 mm, ou même à 1 mm.

3. Article absorbant jetable selon la revendication 2, selon lequel la partie stratifiée (10) comprend une ou deux feuilles stratifiées non tissées qui ont une masse surfacique de 22 g/m² ou moins, de préférence 20 g/m², 17 g/m², ou 13 g/m², ayant de préférence une ou deux feuilles stratifiées non tissées chacune avec une masse surfacique de 17 g/m² ou moins.

4. Article absorbant jetable selon l'une quelconque des revendications 1 à 3, selon lequel ledit matériau élastique (12) est fixé à la feuille de dessus (20) en utilisant des zones d'attachement (14), comprenant typiquement un adhésif, lesdites zones d'attachement (14) étant sous la forme d'un motif en oméga appliqué avec un attachement transversal qui est des parties de boucle, ou sous la forme de zones d'attachement intermittentes à bandes transversales (14), selon lequel à l'état complètement étiré Ls, la distance moyenne entre les zones d'attachement transversales voisines ou parties de zone (14) est comprise entre 0,3 et 2,2 mm, ou entre 0,6 et 1,2 mm.

5. Article absorbant jetable selon la revendication 4, selon lequel la largeur moyenne des zones d'attachement (14) (dans la direction y ; à l'état complètement étiré) est comprise entre 0,3 mm et 0,8 mm, de préférence 0,5 mm ou moins, et/ou le rapport entre ladite largeur moyenne et ladite distance moyenne va de 4:10 à 8:10 ou 6:10, ou de préférence 1:2.

6. Article absorbant jetable selon l'une quelconque des revendications précédentes, selon lequel au moins la surface faisant face au corps de ladite feuille de dessus (20) et/ou ladite partie stratifiée élastique (10) est hydrophobe, ayant de préférence une énergie de surface de moins de 30 dynes/cm, ou même moins de 27 dynes/cm.

7. Article absorbant jetable selon l'une quelconque des revendications précédentes, selon lequel la feuille de dessus (20) a un profil de force tel qu'il a une première force en charge à 200 % d'allongement de 1,6 N ou moins, et une deuxième force à vide à 200 % d'allongement de 0,5 N ou plus.

8. Article absorbant jetable selon l'une quelconque des revendications précédentes, qui est une couche ou un vêtement pour l'incontinence chez l'adulte, selon lequel ladite ouverture est une ouverture de fente d'allongement, et selon lequel la feuille de dessus (20) comprend au moins deux parties stratifiées élastiques (10), qui sont latéralement opposées et s'étendent le long des bords longitudinaux de l'ouverture de telle sorte qu'au moins une partie stratifiée élastique (10) s'étend le long de chaque bord longitudinal de l'ouverture en direction de ou vers le bord transversal avant et/ou le bord transversal arrière de la couche ou du vêtement.
